# EUROPÄISCHE PATENTANMELDUNG

(11) **EP 4 826 408 A1**
(43) Veröffentlichungstag der Anmeldung: **21.10.2026**
(21) Anmeldenummer: 25170422.7
(22) Anmeldetag: 14.04.2025
(51) Int. Cl.: G01R 33/56, A61B 5/055, G06N 3/084, G06T 7/00, G06T 5/50, A61K 49/10, G06T 5/10

(54) **ERZEUGEN VON SYNTHETISCHEN RADIOLOGISCHEN AUFNAHMEN**

(71) Anmelder: Bayer Aktiengesellschaft, 51373 Leverkusen (DE)
(72) Erfinder: BOLZ, Andreas, 51373 Leverkuse (DE)
(74) Vertreter: BIP Patents

(57) **Zusammenfassung**

Die vorliegende Offenbarung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage einer synthetischen Repräsentation einer Leber oder eines Teils einer Leber eines Menschen in einer hepatobiliären Phase nach Applikation eines hepatobiliären Kontrastmittels.

## Beschreibung

### TECHNISCHES GEBIET

Die vorliegende Offenbarung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage einer synthetischen Repräsentation einer Leber oder eines Teils einer Leber eines Menschen in einer hepatobiliären Phase nach Applikation eines hepatobiliären Kontrastmittels.

### EINLEITUNG

Die zeitliche Verfolgung von Vorgängen innerhalb des Körpers eines Menschen mit bildgebenden Verfahren spielt unter anderem bei der Diagnose und/oder Therapie von Krankheiten eine wichtige Rolle.

Als ein Beispiel sei die Detektion und Differentialdiagnose fokaler Leberläsionen mittels dynamischer kontrastverstärkender Magnetresonanztomographie (MRT) mit einem hepatobiliären Kontrastmittel genannt.

Ein hepatobiliäres Kontrastmittel wie beispielsweise Primovist^{®} kann zur Detektion von Tumoren in der Leber eingesetzt werden. Die Blutversorgung des gesunden Lebergewebes erfolgt in erster Linie über die Pfortader (Vena portae), während die Leberarterie (Arteria hepatica) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist^{®} kann zur Erkennung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Bei der durch Primovist^{®} erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist^{®} 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Die zeitliche Verfolgung der Verteilung des Kontrastmittels bietet also eine gute Möglichkeit der Detektion und Differentialdiagnose fokaler Leberläsionen; allerdings zieht sich die Untersuchung über eine vergleichsweise lange Zeitspanne hin. Über diese Zeitspanne sollten Bewegungen des Patienten weitgehend vermieden werden, um Bewegungsartefakte in den MRT-Aufnahmen zu minimieren. Die lang andauernde Bewegungseinschränkung kann für einen Patienten unangenehm sein.

In der Offenlegungsschrift WO2021/052896A1 wird vorgeschlagen, eine oder mehrere MRT-Aufnahmen während der hepatobiliären Phase nicht messtechnisch zu erzeugen, sondern auf Basis von MRT-Aufnahmen aus einer oder mehreren vorangegangenen Phasen zu berechnen (vorherzusagen), um die Aufenthaltsdauer des Patienten im MRT-Scanner zu verkürzen.

In dem in der Offenlegungsschrift WO2021/052896A1 beschriebenen Ansatz wird ein Modell des maschinellen Lernens trainiert, auf Basis von MRT-Aufnahmen eines Untersuchungsbereichs vor und/oder unmittelbar nach der Applikation eines Kontrastmittels eine MRT-Aufnahme des Untersuchungsbereichs zu einem späteren Zeitpunkt vorherzusagen.

Es besteht der Bedarf, die Qualität der vorhergesagten (synthetischen) MRT-Aufnahmen weiter zu verbessern, um einen Radiologen und/oder Arzt bei der Diagnostik und/oder Therapieplanung bestmöglich zu unterstützen.

### ZUSAMMENFASSUNG

Dieser Aufgabe und weiteren Aufgaben widmet sich die vorliegende Offenbarung.

Ein erster Gegenstand der vorliegenden Offenbarung ist ein Computer-implementiertes Verfahren umfassend die Schritte:
- Bereitstellen einer ersten Repräsentation, wobei die erste Repräsentation eine Leber oder einen Teil einer Leber eines Menschen in einer portalvenösen Phase nach Applikation eines hepatobiliären Kontrastmittels im Frequenzraum repräsentiert,
- Bereitstellen einer zweiten Repräsentation, wobei die zweite Repräsentation die Leber oder den Teil der Leber des Menschen in einer Übergangsphase nach Applikation des hepatobiliären Kontrastmittels im Frequenzraum repräsentiert,
- Erzeugen einer ersten Differenzrepräsentation auf Basis der ersten Repräsentation und der zweiten Repräsentation, wobei die erste Differenzrepräsentation eine Differenz zwischen der ersten Repräsentation und der zweiten Repräsentation repräsentiert,
- Eingeben der ersten Differenzrepräsentation in ein trainiertes Modell des maschinellen Lernens,
- Empfangen einer zweiten Differenzrepräsentation als Ausgabe von dem Modell des maschinellen Lernens,
- Erzeugen einer dritten Repräsentation, wobei das Erzeugen der dritten Repräsentation ein Addieren der zweiten Differenzrepräsentation zu der zweiten Repräsentation oder zu der ersten Repräsentation umfasst,
- Transformieren der dritten Repräsentation in eine dritte Repräsentation im Ortsraum,
- Ausgeben und/oder Speichern der dritten Repräsentation im Ortsraum und/oder Übermitteln der dritten Repräsentation im Ortsraum an ein separates Computersystem,
wobei das trainierte Modell des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation als Eingabedaten und (ii) eine zweite Referenz-Differenzrepräsentation als Zieldaten umfassen, wobei die erste Referenz-Differenzrepräsentation eine Differenz einer ersten Referenz-Repräsentation und einer zweiten Referenz-Repräsentation repräsentiert und die zweite Referenz-Differenzrepräsentation eine Differenz der zweiten Referenz-Repräsentation und einer dritten Referenz-Repräsentation oder der ersten Referenz-Repräsentation und der dritten Referenz-Repräsentation repräsentiert, wobei die erste Referenz-Repräsentation eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation die Leber oder den Teil der Leber der Referenzperson in einer Übergangsphase nach Applikation des hepatobiliären Kontrastmittels im Frequenzraum repräsentiert und die dritte Referenz-Repräsentation die Leber oder den Teil der Leber der Referenzperson in einer hepatobiliären Phase nach Applikation des hepatobiliären Kontrastmittels im Frequenzraum repräsentiert.

Ein weiterer Gegenstand der vorliegenden Offenbarung ist ein Computersystem umfassend
- eine Empfangseinheit,
- eine Steuer- und Recheneinheit und
- eine Ausgabeeinheit,
wobei die Steuer- und Recheneinheit konfiguriert ist,
- eine erste Repräsentation bereitzustellen oder die Empfangseinheit zu veranlassen, die erste Repräsentation zu empfangen, wobei die erste Repräsentation eine Leber oder einen Teil einer Leber eines Menschen in einer portalvenösen Phase nach Applikation eines hepatobiliären Kontrastmittels im Frequenzraum repräsentiert,
- eine zweite Repräsentation bereitzustellen oder die Empfangseinheit zu veranlassen, die zweite Repräsentation zu empfangen, wobei die zweite Repräsentation die Leber oder den Teil der Leber des Menschen in einer Übergangsphase nach Applikation des hepatobiliären Kontrastmittels im Frequenzraum repräsentiert,
- eine erste Differenzrepräsentation auf Basis der ersten Repräsentation und der zweiten Repräsentation zu erzeugen, wobei die erste Differenzrepräsentation eine Differenz zwischen der ersten Repräsentation und der zweiten Repräsentation repräsentiert,
- die erste Differenzrepräsentation in ein trainiertes Modell des maschinellen Lernens einzugeben,
- eine zweite Differenzrepräsentation als Ausgabe von dem Modell des maschinellen Lernens zu empfangen,
- eine dritte Repräsentation zu erzeugen, wobei das Erzeugen der dritten Repräsentation ein Addieren der zweiten Differenzrepräsentation zu der zweiten Repräsentation oder zu der ersten Repräsentation umfasst,
- die dritte Repräsentation in eine dritte Repräsentation im Ortsraum zu transformieren,
- die Ausgabeeinheit zu veranlassen, die dritte Repräsentation im Ortsraum auszugeben, in einem Datenspeicher zu speichern und/oder an ein separates Computersystem zu übermitteln,
wobei das trainierte Modell des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation als Eingabedaten und (ii) eine zweite Referenz-Differenzrepräsentation als Zieldaten umfassen, wobei die erste Referenz-Differenzrepräsentation eine Differenz einer ersten Referenz-Repräsentation und einer zweiten Referenz-Repräsentation repräsentiert und die zweite Referenz-Differenzrepräsentation eine Differenz der zweiten Referenz-Repräsentation und einer dritten Referenz-Repräsentation oder der ersten Referenz-Repräsentation und der dritten Referenz-Repräsentation repräsentiert, wobei die erste Referenz-Repräsentation eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation die Leber oder den Teil der Leber der Referenzperson in einer Übergangsphase nach Applikation des hepatobiliären Kontrastmittels im Frequenzraum repräsentiert und die dritte Referenz-Repräsentation die Leber oder den Teil der Leber der Referenzperson in einer hepatobiliären Phase nach Applikation des hepatobiliären Kontrastmittels im Frequenzraum repräsentiert.

Ein weiterer Gegenstand der vorliegenden Offenbarung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Bereitstellen einer ersten Repräsentation, wobei die erste Repräsentation eine Leber oder einen Teil einer Leber eines Menschen in einer portalvenösen Phase nach Applikation eines hepatobiliären Kontrastmittels im Frequenzraum repräsentiert,
- Bereitstellen einer zweiten Repräsentation, wobei die zweite Repräsentation die Leber oder den Teil der Leber des Menschen in einer Übergangsphase nach Applikation des hepatobiliären Kontrastmittels im Frequenzraum repräsentiert,
- Erzeugen einer ersten Differenzrepräsentation auf Basis der ersten Repräsentation und der zweiten Repräsentation, wobei die erste Differenzrepräsentation eine Differenz zwischen der ersten Repräsentation und der zweiten Repräsentation repräsentiert,
- Eingeben der ersten Differenzrepräsentation in ein trainiertes Modell des maschinellen Lernens,
- Empfangen einer zweiten Differenzrepräsentation als Ausgabe von dem Modell des maschinellen Lernens,
- Erzeugen einer dritten Repräsentation, wobei das Erzeugen der dritten Repräsentation ein Addieren der zweiten Differenzrepräsentation zu der zweiten Repräsentation oder zu der ersten Repräsentation umfasst,
- Transformieren der dritten Repräsentation in eine dritte Repräsentation im Ortsraum,
- Ausgeben und/oder Speichern der dritten Repräsentation im Ortsraum und/oder Übermitteln der dritten Repräsentation im Ortsraum an ein separates Computersystem,
wobei das trainierte Modell des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation als Eingabedaten und (ii) eine zweite Referenz-Differenzrepräsentation als Zieldaten umfassen, wobei die erste Referenz-Differenzrepräsentation eine Differenz einer ersten Referenz-Repräsentation und einer zweiten Referenz-Repräsentation repräsentiert und die zweite Referenz-Differenzrepräsentation eine Differenz der zweiten Referenz-Repräsentation und einer dritten Referenz-Repräsentation oder der ersten Referenz-Repräsentation und der dritten Referenz-Repräsentation repräsentiert, wobei die erste Referenz-Repräsentation eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation die Leber oder den Teil der Leber der Referenzperson in einer Übergangsphase nach Applikation des hepatobiliären Kontrastmittels im Frequenzraum repräsentiert und die dritte Referenz-Repräsentation die Leber oder den Teil der Leber der Referenzperson in einer hepatobiliären Phase nach Applikation des hepatobiliären Kontrastmittels im Frequenzraum repräsentiert.

Ein weiterer Gegenstand der vorliegenden Offenbarung ist ein Kit umfassend ein hepatobiliäres Kontrastmittel und ein Computerprogrammprodukt umfassend ein Computerprogramm, das in einen Arbeitsspeicher eines Computersystems geladen werden kann und dort das Computersystem dazu veranlasst, folgende Schritte ausführen:
- Bereitstellen einer ersten Repräsentation, wobei die erste Repräsentation eine Leber oder einen Teil einer Leber eines Menschen in einer portalvenösen Phase nach Applikation des hepatobiliären Kontrastmittels im Frequenzraum repräsentiert,
- Bereitstellen einer zweiten Repräsentation, wobei die zweite Repräsentation die Leber oder den Teil der Leber des Menschen in einer Übergangsphase nach Applikation des hepatobiliären Kontrastmittels im Frequenzraum repräsentiert,
- Erzeugen einer ersten Differenzrepräsentation auf Basis der ersten Repräsentation und der zweiten Repräsentation, wobei die erste Differenzrepräsentation eine Differenz zwischen der ersten Repräsentation und der zweiten Repräsentation repräsentiert,
- Eingeben der ersten Differenzrepräsentation in ein trainiertes Modell des maschinellen Lernens,
- Empfangen einer zweiten Differenzrepräsentation als Ausgabe von dem Modell des maschinellen Lernens,
- Erzeugen einer dritten Repräsentation, wobei das Erzeugen der dritten Repräsentation ein Addieren der zweiten Differenzrepräsentation zu der zweiten Repräsentation oder zu der ersten Repräsentation umfasst,
- Transformieren der dritten Repräsentation in eine dritte Repräsentation im Ortsraum,
- Ausgeben und/oder Speichern der dritten Repräsentation im Ortsraum und/oder Übermitteln der dritten Repräsentation im Ortsraum an ein separates Computersystem,
wobei das trainierte Modell des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation als Eingabedaten und (ii) eine zweite Referenz-Differenzrepräsentation als Zieldaten umfassen, wobei die erste Referenz-Differenzrepräsentation eine Differenz einer ersten Referenz-Repräsentation und einer zweiten Referenz-Repräsentation repräsentiert und die zweite Referenz-Differenzrepräsentation eine Differenz der zweiten Referenz-Repräsentation und einer dritten Referenz-Repräsentation oder der ersten Referenz-Repräsentation und der dritten Referenz-Repräsentation repräsentiert, wobei die erste Referenz-Repräsentation eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation die Leber oder den Teil der Leber der Referenzperson in einer Übergangsphase nach Applikation des hepatobiliären Kontrastmittels im Frequenzraum repräsentiert und die dritte Referenz-Repräsentation die Leber oder den Teil der Leber der Referenzperson in einer hepatobiliären Phase nach Applikation des hepatobiliären Kontrastmittels im Frequenzraum repräsentiert.

### KURZE BESCHREIBUNG DER ZEICHNUNGEN

Fig. 1 zeigt eine Ausführungsform des computer-implementierten Verfahrens der vorliegenden Offenbarung in Form eines Ablaufschemas.
Fig. 2 zeigt beispielhaft und schematisch ein Verfahren zum Trainieren des Modells des maschinellen Lernens der vorliegenden Offenbarung.
Fig. 3 zeigt beispielhaft und schematisch die Verwendung des trainierten Modells des maschinellen Lernens zur Vorhersage.
Fig. 4 zeigt beispielhaft und schematisch ein Computersystem gemäß der vorliegenden Offenbarung, das zum Trainieren des Modells des maschinellen Lernens und/oder zur Verwendung des trainierten Modells des maschinellen Lernens zur Vorhersage verwendet werden kann.
Fig. 5 zeigt beispielhaft und schematisch eine weitere Ausführungsform des erfindungsgemäßen Computersystems.

### AUSFÜHRLICHE BESCHREIBUNG

Die Erfindung wird nachstehend näher erläutert, ohne zwischen den Erfindungsgegenständen (Verfahren, Computersystem, Computerprogrammprodukt, Kit) zu unterscheiden. Vielmehr sollen die folgenden Ausführungen sinngemäß für alle Gegenstände der Erfindung (Verfahren, Computersystem, Computerprogrammprodukt, Kit) gelten, unabhängig davon, in welchem Zusammenhang sie gemacht werden.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Mit Hilfe der vorliegenden Erfindung können Repräsentationen einer Leber oder eines Teils einer Leber eines Menschen vorhergesagt werden. Eine solche vorhergesagte Repräsentation wird in dieser Offenbarung auch als synthetische Repräsentation bezeichnet. Der Begriff "synthetisch" bedeutet, dass die Repräsentation nicht das (direkte) Ergebnis einer physikalischen Messung an einem realen Untersuchungsobjekt (z.B. einem Menschen) ist, sondern dass die synthetische Repräsentation mit Hilfe eines Modells des maschinellen Lernens erzeugt wird. Ein Synonym für den Begriff "synthetisch" ist der Begriff "künstlich". Die synthetische Repräsentation basiert jedoch auf einer oder mehreren gemessenen Repräsentationen, d.h., das Modell des maschinellen Lernens kann so konfiguriert und trainiert werden, dass es eine synthetische Repräsentation auf der Grundlage eines oder mehrerer gemessener Repräsentationen (und/oder optional weiteren Daten) erzeugt.

Eine Repräsentation einer Leber oder eines Teils einer Leber ist üblicherweise das Ergebnis einer radiologischen Untersuchung. Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonographie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Offenbarung umfasst damit insbesondere die folgenden Untersuchungsmethoden: Computertomographie (CT), Magnetresonanztomographie (MRT), Sonographie, Positronenemissionstomographie (PET).

In einer Ausführungsform der vorliegenden Offenbarung handelt es sich bei der radiologischen Untersuchung um eine magnetresonanztomographische oder computertomographische Untersuchung.

In einer Ausführungsform der vorliegenden Offenbarung handelt es sich bei der radiologischen Untersuchung um eine magnetresonanztomographische Untersuchung.

Die Magnetresonanztomographie, abgekürzt MRT (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MRT-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MRT-Daten liegen zunächst als Rohdaten im Frequenzraum vor (so genannte k-Raum-Daten), und können durch anschließende inverse Fourier-Transformation in den Ortsraum (Bildraum) transformiert werden.

Eine Repräsentation im Sinne der vorliegenden Offenbarung kann eine MRT-Aufnahme, eine CT-Aufnahme, ein Ultraschallbild, eine PET-Aufnahme oder dergleichen sein oder die Repräsentation kann aus einer oder mehreren MRT-Aufnahmen, CT-Aufnahmen, PET-Aufnahme, Ultraschallbildern oder dergleichen erzeugt werden.

Bei radiologischen Untersuchungen werden häufig Kontrastmittel zur Kontrastverstärkung eingesetzt. "Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate) als Kontrastmittel verwendet. Im Fall der Sonografie werden üblicherweise Flüssigkeiten, die gasgefüllte Mikrobläschen enthalten, intravenös verabreicht. In der Positronenemissionstomographie werden üblicherweise Radiotracer eingesetzt. Radiotracer enthalten ein radioaktives Isotop und emittieren Positronen während ihres Zerfalls. Diese Positronen interagieren mit Elektronen im Körper, was zur Emission von Gammastrahlen führt, die vom PET-Scanner detektiert werden können.

MRT-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: superparamagnetic iron oxide). Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}), Gadobutrol (Gadovist^{®}) und Gadoxetsäure (Primovist^{®}/Eovist^{®}).

In einem ersten Schritt werden eine erste Repräsentation und eine zweite Repräsentation bereitgestellt.

Die erste Repräsentation repräsentiert eine Leber oder einen Teil einer Leber eines Menschen in einer portalvenösen Phase nach Applikation eines hepatobiliären Kontrastmittels im Frequenzraum.

Die zweite Repräsentation repräsentiert die Leber oder den Teil der Leber des Menschen in einer Übergangsphase nach Applikation des hepatobiliären Kontrastmittels im Frequenzraum.

Ein hepatobiliäres Kontrastmittel zeichnet sich üblicherweise dadurch aus, dass es anteilig spezifisch von Leberzellen, den Hepatozyten, aufgenommen wird, sich im Funktionsgewebe (Parenchym) anreichert und die Kontraste in gesundem Lebergewebe verstärkt, bevor es anschließend über Galle in die Faeces ausgeschieden werden. Beispiele für derartige Kontrastmittel auf der Basis der Gadoxetsäure sind in US 6,039,931A beschrieben; sie sind kommerziell zum Beispiel unter den Markennamen Primovist^{®} und Eovist^{®} erhältlich. Ein weiteres MRT-Kontrastmittel mit einer geringeren Aufnahme in die Hepatozyten ist Gadobenate Dimeglumine (Multihance^{®}). Weitere hepatobiliäre Kontrastmittel werden unter anderem in WO2022/194777 beschrieben.

Gadoxetat-Dinatrium (GD, Primovist^{®}) ist zur Verwendung in der MRT der Leber zugelassen, um Läsionen bei Patienten mit bekannter oder vermuteter fokaler Lebererkrankung zu erkennen und zu charakterisieren. GD weist mit seiner lipophilen Ethoxybenzyl-Einheit eine zweiphasige Verteilung auf: zunächst eine Verteilung im intravaskulären und interstitiellen Raum nach Bolusinjektion, gefolgt von einer selektiven Aufnahme durch Hepatozyten. GD wird über die Nieren und den hepatobiliären Weg (50:50 dualer Ausscheidungsmechanismus) in etwa gleichen Mengen unverändert aus dem Körper ausgeschieden. GD wird aufgrund seiner selektiven Anreicherung im gesunden Lebergewebe auch als hepatobiliäres Kontrastmittel bezeichnet.

GD ist in einer Dosis von 0,1 ml / kg Körpergewicht (BW) (0,025 mmol / kg BW Gd) zugelassen. Die empfohlene Verabreichung von GD umfasst eine unverdünnte intravenöse Bolusinjektion mit einer Flussrate von ungefähr 2 ml / Sekunde, gefolgt von einer Spülung der i.v. Kanüle mit einer physiologischen Kochsalzlösung. Ein Standardprotokoll für die Leberbildgebung unter Verwendung von GD besteht aus mehreren Planungs- und Vorkontrastsequenzen. Nach i.v. Bolusinjektion des Kontrastmittels, werden üblicherweise dynamische Aufnahmen während der arteriellen (ungefähr 30 Sekunden nach der Injektion, p.i.), portalvenösen (ungefähr 60 Sekunden p.i.) und der Übergangsphase (ungefähr 2-5 Minuten p.i.) aufgenommen. Die Übergangsphase zeigt typischerweise bereits einen gewissen Anstieg der Lebersignalintensität aufgrund der beginnenden Aufnahme des Mittels in Hepatozyten.

In einer Ausführungsform handelt es sich bei dem hepatobiliären Kontrastmittel um Gadoxetat-Dinatrium.

In einer Ausführungsform der vorliegenden Offenbarung handelt es sich bei dem hepatobiliären Kontrastmittel um ein Mittel, das einen Gd³⁺-Komplex einer Verbindung der Formel (I) 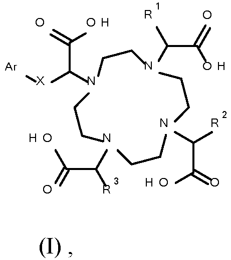 umfasst, wobei
Ar eine Gruppe ausgewählt aus 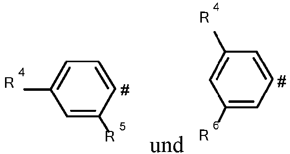 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-# ausgewählt wird,
wobei * die Anknüpfung zu Ar darstellt und # die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H;C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
   und
R⁶ ein Wasserstoffatom darstellt,
oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen Gd³⁺-Komplex einer Verbindung der Formel (II) 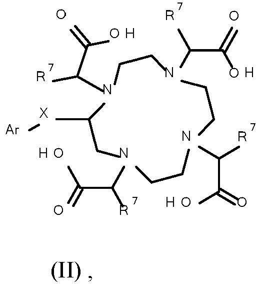 umfasst, wobei
Ar eine Gruppe ausgewählt aus 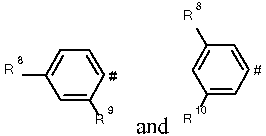 darstellt,
wobei # die Anknüpfung zu X darstellt,
X eine Gruppe darstellt, die aus CH₂, (CH₂)₂, (CH₂)₃, (CH₂)₄ and *-(CH₂)₂-O-CH₂-# ausgewählt wird, wobei * die Anknüpfung zu Ar darstellt und # die Anknüpfung zum Essigsäurerest darstellt,
R⁷ ein Wasserstoffatom oder eine Gruppe ausgewählt aus C₁-C₃-Alkyl, -CH₂OH,
-(CH₂)₂OH und -CH₂OCH₃ darstellt;
R⁸ eine Gruppe ausgewählt aus
C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.

Der Begriff "C₁-C₃-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "C₂-C₄-Alkyl" bedeutet eine lineare oder verzweigte, gesättigte, einwertige Kohlenwasserstoffgruppe mit 2, 3 oder 4 Kohlenstoffatomen.

Der Begriff "C₂-C₄-Alkoxy" bedeutet eine lineare oder verzweigte, gesättigte, einwertige Gruppe der Formel (C₂-C₄-Alkyl)-O-, in der der Begriff "C₂-C₄-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

Die erste Repräsentation repräsentiert eine Leber oder einen Teil einer Leber eines Menschen in einer portalvenösen Phase nach Applikation eines hepatobiliären Kontrastmittels. Die zweite Repräsentation repräsentiert die Leber oder den Teil der Leber des Menschen in einer portalvenösen Phase nach Applikation des hepatobiliären Kontrastmittels.

Das hepatobiliäre Kontrastmittel wird üblicherweise intravenös z.B. in eine Armvene des Menschen verabreicht. Nach der intravenösen Applikation des hepatobiliären Kontrastmittels in Form eines Bolus in eine Armvene erreicht das Kontrastmittel die Leber zunächst über die Arterien. Diese sind in den entsprechenden radiologischen Aufnahmen kontrastverstärkt dargestellt. Die Phase, in der die Leberarterien in den radiologischen Aufnahmen kontrastverstärkt dargestellt sind, wird als "arterielle Phase" bezeichnet.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in radiologischen Aufnahmen kontrastverstärkt dargestellt sind, wird als "portalvenöse Phase" bezeichnet. Diese Phase kann bereits während der arteriellen Phase beginnen und sich mit dieser überlagern.

An die portalvenöse Phase schließt sich die "Übergangsphase" (engl. *transitional phase*) an, in der der Kontrast in den Leberarterien weiter absinkt, der Kontrast in den Lebervenen ebenfalls absinkt. Bei Verwendung eines hepatobiliären Kontrastmittels steigt der Kontrast in den gesunden Leberzellen in der Übergangsphase allmählich an.

Die arterielle Phase, die portalvenöse Phase und die Übergangsphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Die erste Repräsentation und die zweite Repräsentation repräsentieren eine Leber oder einen Teil einer Leber eines Menschen im Frequenzraum. Die erste Repräsentation und die zweite Repräsentation können beispielsweise durch Fourier-Transformation aus den entsprechenden Ortsraum-Repräsentationen gewonnen werden.

In einer Ortsraum-Repräsentation wird der Untersuchungsbereich (z.B. eine Leber oder ein Teil einer Leber eines Menschen) üblicherweise durch eine Vielzahl an Bildelementen (z.B. Pixel oder Voxel) repräsentiert, die beispielsweise rasterförmig angeordnet sein können, wobei jedes Bildelement einen Teil des Untersuchungsbereichs repräsentiert. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

In einer Repräsentation im Frequenzraum, in dieser Beschreibung auch als Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/1softaee261/book-fall-07.pdf.

Die erste Repräsentation kann eine MRT-Aufnahme einer Leber oder eines Teils einer Leber eines Menschen nach Applikation eines hepatobiliären MRT-Kontrastmittels sein oder umfassen oder aus einer solchen gewonnen werden (z.B. durch Fourier-Transformation auf Basis einer entsprechenden Ortsraum-Repräsentation).

Die erste Repräsentation kann eine CT-Aufnahme einer Leber oder eines Teils einer Leber eines Menschen nach Applikation eines hepatobiliären MRT-Kontrastmittels sein oder umfassen oder aus einer solchen gewonnen werden (z.B. durch Fourier-Transformation auf Basis einer entsprechenden Ortsraum-Repräsentation).

Ebenso kann auch die zweite Repräsentation eine MRT-Aufnahme oder eine CT-Aufnahme einer Leber oder eines Teils einer Leber eines Menschen nach Applikation eines hepatobiliären MRT-Kontrastmittels sein oder umfassen oder aus einer solchen gewonnen werden (z.B. durch Fourier-Transformation auf Basis einer entsprechenden Ortsraum-Repräsentation).

In einem weiteren Schritt wird eine erste Differenzrepräsentation auf Basis der ersten Repräsentation und der zweiten Repräsentation erzeugt. Die erste Differenzrepräsentation repräsentiert eine Differenz zwischen der ersten Repräsentation und der zweiten Repräsentation. Mit anderen Worten: die erste Differenzrepräsentation repräsentiert Unterschiede zwischen der ersten Repräsentation und der zweiten Repräsentation. Das Erzeugen der ersten Differenzrepräsentation kann beispielsweise ein Subtrahieren der ersten Repräsentation von der zweiten Repräsentation umfassen. Das Erzeugen der ersten Differenzrepräsentation kann beispielsweise ein Subtrahieren der zweiten Repräsentation von der ersten Repräsentation umfassen.

Grundsätzlich ist es auch möglich, Unterschiede zwischen der ersten Repräsentation und der zweiten Repräsentation nicht durch Subtraktion zu erfassen, sondern beispielsweise durch Division. Zur Vereinfachung werden die Aspekte der vorliegenden Offenbarung anhand von Differenzen beschrieben, ohne die Offenbarung jedoch auf diese Ausführungsform beschränken zu wollen. Dem Fachmann der Bildverarbeitung ist bewusst, dass Unterschiede zwischen bildhaften Repräsentationen auch anders als durch Subtraktion (z.B. durch Division) ermittelt und ausgedrückt werden können. Damit sollen auch andere Verfahren und Repräsentationen, die Unterschiede zwischen zwei Repräsentationen erfassen und repräsentieren, in den Äquivalenzbereich der vorliegenden Offenbarung fallen.

Es ist möglich, dass die erste und/oder die zweite Repräsentation und/oder die erste Differenzrepräsentation mittels eines oder mehrerer Filter gefiltert werden. Beispiele solcher Filter sind frequenzabhängige Gewichtungen wie sie beispielsweise in WO2024100234A1 oder EP24182725.2 beschrieben sind. Aber auch andere Filter sind denkbar.

Die erste Differenzrepräsentation wird in ein trainiertes Modell des maschinellen Lernens eingegeben. Das Modell des maschinellen Lernens ist konfiguriert und wurde trainiert, auf Basis einer ersten Differenzrepräsentation und Modellparametern (und optional weiterer Eingabedaten) eine zweite Differenzrepräsentation zu erzeugen.

Die zweite Differenzrepräsentation ist eine von dem trainierten Modell des maschinellen Lernens auf Basis der ersten Differenzrepräsentation (und optional weiteren Eingabedaten) vorhergesagte Differenzrepräsentation. Die zweite Differenzrepräsentation ist eine synthetische Repräsentation. Die zweite Differenzrepräsentation repräsentiert eine Differenz (Unterschiede) zwischen der zweiten Repräsentation der Leber oder des Teils der Leber des Menschen und einer dritten Repräsentation oder zwischen der ersten Repräsentation der Leber oder des Teils der Leber des Menschen und einer dritten Repräsentation (je nachdem, was trainiert wurde). Die dritte Repräsentation ist eine Repräsentation der Leber oder des Teils der Leber des Menschen in einer hepatobiliären Phasen nach Applikation des hepatobiliären Kontrastmittels im Frequenzraum. Die dritte Repräsentation ist eine hypothetische oder theoretische oder angenommene Repräsentation, denn sie wurde, im Gegensatz zu der ersten Repräsentation und der zweiten Repräsentation nicht gemessen. Stattdessen wird sie mit Hilfe der zweiten Differenzrepräsentation, die ebenfalls nicht gemessen, sondern vorhergesagt wurde, erzeugt (wie weiter unten beschrieben ist).

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modellparametern liefern. Das Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function*) quantifiziert werden. Eine solche Fehlerfunktion kann verwendet werden, um einen Fehler (engl.: *loss*) für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall umfassen die Trainingsdaten zum Trainieren des Modells des maschinellen Lernens, für jede Referenzperson einer Vielzahl an Referenzpersonen, (i) eine erste Referenz-Differenzrepräsentation als Eingabedaten und (ii) eine zweite Referenz-Differenzrepräsentation als Zieldaten.

Der Begriff Vielzahl bedeutet üblicherweise mehr als 10, z.B. 100 oder mehr als 500.

Die erste Referenz-Differenzrepräsentation repräsentiert eine Differenz (Unterschiede) einer ersten Referenz-Repräsentation und einer zweiten Referenz-Repräsentation. Die zweite Referenz-Differenzrepräsentation repräsentiert (i) eine Differenz (Unterschiede) der zweiten Referenz-Repräsentation und einer dritten Referenz-Repräsentation oder (ii) eine Differenz (Unterschiede) der ersten Referenz-Repräsentation und einer dritten Referenz-Repräsentation.

Die erste Referenz-Repräsentation repräsentiert eine Leber oder einen Teil einer Leber der Referenzperson in einer portalvenösen Phase nach Applikation des hepatobiliären Kontrastmittels im Frequenzraum. Die zweite Referenz-Repräsentation repräsentiert die Leber oder den Teil der Leber der Referenzperson in einer Übergangsphase nach Applikation des hepatobiliären Kontrastmittels im Frequenzraum. Die dritte Referenz-Repräsentation repräsentiert die Leber oder den Teil der Leber der Referenzperson in einer hepatobiliären Phase nach Applikation des hepatobiliären Kontrastmittels im Frequenzraum.

Der Begriff "Referenz" wird an einigen Stellen in dieser Offenlegung verwendet, um die Daten, die zum Trainieren und/oder Validieren des Modells des maschinellen Lernens verwendet werden, von den Daten zu unterscheiden, die zum Erzeugen einer synthetischen Repräsentation unter Verwendung des trainierten Modells des maschinellen Lernens verwendet werden. Der Begriff "Referenz" ist jedoch nicht in einem anderen einschränkenden Sinne zu verstehen; diese Unterscheidung dient lediglich dazu, einen Einwand der Unklarheit in Patenterteilungsverfahren zu vermeiden.

Die Referenzperson ist ein Mensch. Die erste Referenz-Repräsentation hat die gleichen Eigenschaften wie die in dieser Offenbarung beschriebene erste Repräsentation. Die zweite Referenz-Repräsentation hat die gleichen Eigenschaften wie die in dieser Offenbarung beschriebene zweite Repräsentation. Die erste Referenz-Differenzrepräsentation hat die gleichen Eigenschaften wie die in dieser Offenbarung beschriebene erste Differenzrepräsentation.

Neben den genannten Eingabedaten können weitere Daten als Eingabedaten zum Trainieren des Modells des maschinellen Lernens (und zur Vorhersage nach dem Training) verwendet werden. Dies können Daten zum Untersuchungsobjekt (dem Menschen) sein, wie beispielsweise Alter, Geschlecht, Körpergröße, Körpergewicht, diagnostizierte Krankheiten, Lebensgewohnheiten (z.B. Alkoholkonsum) und/oder andere/weitere Daten sein. Es können auch weitere Repräsentation der Leber oder des Teils der Leber des Menschen sein, wie beispielsweise eine Repräsentation vor der Applikation des hepatobiliären Kontrastmittels (native Phase).

Das Trainieren des Modells des maschinellen Lernens umfasst üblicherweise die folgenden Schritte: für jede Referenzperson der Vielzahl an Referenzpersonen:
- Eingeben der ersten Referenz-Differenzrepräsentation (und optional weiterer Eingabedaten) in das Modell des maschinellen Lernens,
- Empfangen einer vorhergesagten zweiten Referenz-Differenzrepräsentation als Ausgabe von dem Modell des maschinellen Lernens,
- Quantifizieren einer Abweichung zwischen der zweiten Referenz-Differenzrepräsentation und der vorhergesagten zweiten Referenz-Differenzrepräsentation,
- Reduzieren der Abweichung durch Modifizieren von Modellparametern des Modells des maschinellen Lernens.

Das Modifizieren (Anpassen) der Modellparameter erfolgt üblicherweise in einem Optimierungsverfahren wie beispielsweise einem Gradientenabstiegsverfahren.

Das Training des Modells des maschinellen Lernens kann beendet werden, wenn ein Stoppkriterium erfüllt ist. Ein solches Stoppkriterium kann beispielsweise sein: eine vorab festgelegte maximale Anzahl von Trainingsschritten/-zyklen/-epochen wurde durchgeführt, Abweichungen zwischen Ausgabedaten und Zieldaten können nicht mehr durch Modifizieren der Modellparameter reduziert werden, ein vorab festgelegtes Minimum der Fehlerfunktion wird erreicht und/oder ein Extremwert (z. B. Maximum oder Minimum) eines anderen Leistungswerts wird erreicht.

Beim Training eines maschinellen Lernmodells wird der Trainingsdatensatz in der Regel in mehrere Teilmengen unterteilt, um eine robuste Auswertung und Generalisierung zu gewährleisten. Zu den gängigsten Sätzen gehören ein Trainingssatz, ein Validierungssatz und ein Testsatz. Der Trainingssatz wird zum Trainieren des Modells des maschinellen Lernens verwendet. Der Validierungssatz kann zur Abstimmung von Hyperparametern und zur Entscheidungsfindung bezüglich der Modellarchitektur verwendet werden. Der Validierungssatz kann zudem bei der Bewertung der Leistung des Modells während des Trainings helfen. Der Validierungssatz ermöglicht die Überwachung von Überanpassung und die Vornahme von Anpassungen zur Verbesserung der Modellleistung. Der Testdatensatz wird in der Regel getrennt von den Trainings- und Validierungsdatensätzen aufbewahrt und dient dazu, die endgültige Leistung des Modells nach Abschluss des Trainings zu bewerten. Es gibt verschiedene Aufteilungsstrategien für die Aufteilung der Daten, z. B. zufällige Aufteilung, k-fache Kreuzvalidierung und geschichtete Aufteilung.

Das Modell des maschinellen Lernens kann ein künstliches neuronales Netzwerk sein oder ein solches umfassen.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein oder ein solches umfassen (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netz kann ein *Regularized Generative Adversarial Network* sein (siehe z.B. Q. Li et al.: RegGAN: An End-to-End Network for Building Footprint Generation with Boundary Regularization, Remote Sens. 2022, 14, 1835).

Das künstliche neuronale Netzwerk kann ein *Conditional Adversarial Network* sein oder ein solches umfassen (siehe z.B. P. Isola et al.: Image-to-Image Translation with Conditional Adversarial Networks, arXiv:1611.07004 [cs.CV]).

Das künstliche neuronale Netz kann ein Transformer-Netzwerk sein oder ein solches umfassen (siehe z.B. D. Karimi et al.: Convolution-Free Medical Image Segmentation using Transformers, arXiv:2102.13645 [eess.IV]).

Ist das Modell des maschinellen Lernens trainiert, kann es zur Vorhersage genutzt werden. Es kann genutzt werden, um eine synthetische Repräsentation für eine neue Person zu erzeugen. Der Begriff "neu" bedeutet, dass Repräsentationen der neuen Person üblicherweise nicht im Training verwendet worden sind.

In das trainierte Modell des maschinellen Lernens wird also wie oben bereits beschrieben die erste Differenzrepräsentation (und optional weitere Eingabedaten) eingegeben. Das trainierte Modell des maschinellen Lernens erzeugt auf Basis der ersten Differenzrepräsentation (und optional der weiteren Eingabedaten) eine (vorhergesagte, synthetische) zweite Differenzrepräsentation.

In einem weiteren Schritt wird auf Basis der vom trainierten Modell des maschinellen Lernens erzeugten zweiten Differenzrepräsentation und der bereits vorliegenden zweiten Repräsentation oder ersten Repräsentation eine dritte Repräsentation erzeugt. Das Erzeugen der dritten Repräsentation umfasst üblicherweise ein Addieren der zweiten Differenzrepräsentation zu der zweiten Repräsentation oder ein Addieren der zweiten Differenzrepräsentation zu der ersten Repräsentation.

Die dritte Repräsentation ist eine synthetische Repräsentation, da sie auf der synthetischen zweiten Differenzrepräsentation basiert.

Die dritte Repräsentation repräsentiert die Leber oder den Teil der Leber des Menschen in einer hepatobiliären Phase nach Applikation des hepatobiliären Kontrastmittels im Frequenzraum.

In einem weiteren Schritt kann auf Basis der dritten Frequenzraum-Repräsentation eine entsprechende dritte Ortsaum-Repräsentation erzeugt werden. Die dritte Ortsraum-Repräsentation kann beispielsweise durch inverse Fourier-Transformation aus der dritten Frequenzraum-Repräsentation erzeugt werden. Die dritte Ortsraum-Repräsentation ist eine synthetische Repräsentation, da sie auf der synthetischen zweiten Differenzrepräsentation basiert. Die dritte Ortsraum-Repräsentation repräsentiert die Leber oder den Teil der Leber des Menschen in der hepatobiliären Phase nach Applikation des hepatobiliären Kontrastmittels im Ortsraum.

Die dritte Ortsraum-Repräsentation kann ausgegeben (z.B. auf einem Monitor angezeigt und/oder auf einem Drucker ausgedruckt), in einem Datenspeicher gespeichert und/oder an ein separates Computersystem übermittelt werden.

Das Erzeugen einer synthetischen Repräsentation der Leber oder eines Teils der Leber eines Menschen in einer hepatobiliären Phase nach Applikation eines hepatobiliären Kontrastmittels hat den Vorteil, dass die für den Menschen anstrengende radiologische Untersuchung auf die portalvenöse und die Übergangsphase beschränkt werden kann. Der Mensch verbringt also z.B. weniger Zeit im Tomographen. Die Qualität der synthetischen Repräsentation ist höher im Vergleich zu synthetischen Repräsentationen, die durch andere Verfahren erzeugt werden.

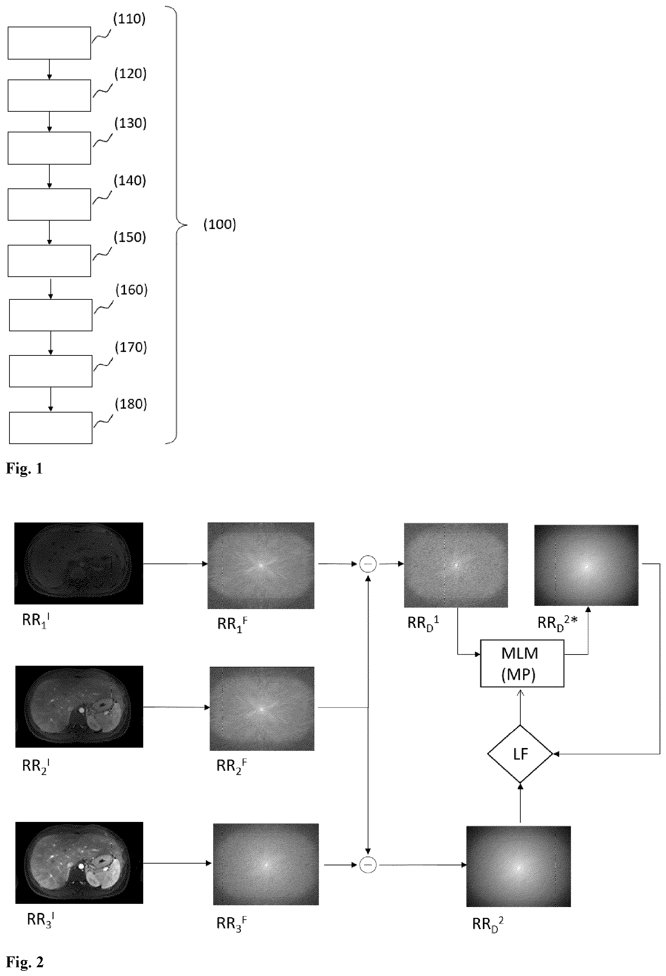

Fig. 1 zeigt eine Ausführungsform des computer-implementierten Verfahrens der vorliegenden Offenbarung in Form eines Ablaufschemas.

Das Verfahren (100) umfasst die Schritte:

| | |
|---|---|
| (110) | Bereitstellen einer ersten Repräsentation, wobei die erste Repräsentation eine Leber oder einen Teil einer Leber eines Menschen in einer portalvenösen Phase nach Applikation eines hepatobiliären Kontrastmittels im Frequenzraum repräsentiert, |
| (120) | Bereitstellen einer zweiten Repräsentation, wobei die zweite Repräsentation die Leber oder den Teil der Leber des Menschen in einer Übergangsphase nach Applikation des hepatobiliären Kontrastmittels im Frequenzraum repräsentiert, |
| (130) | Erzeugen einer ersten Differenzrepräsentation auf Basis der ersten Repräsentation und der zweiten Repräsentation, wobei die erste Differenzrepräsentation eine Differenz zwischen der ersten Repräsentation und der zweiten Repräsentation repräsentiert, |
| (140) | Eingeben der ersten Differenzrepräsentation in ein trainiertes Modell des maschinellen Lernens, |
| (150) | Empfangen einer zweiten Differenzrepräsentation als Ausgabe von dem Modell des maschinellen Lernens, |
| (160) | Erzeugen einer dritten Repräsentation, wobei das Erzeugen der dritten Repräsentation ein Addieren der zweiten Differenzrepräsentation zu der zweiten Repräsentation oder zu der ersten Repräsentation umfasst, |
| (170) | Transformieren der dritten Repräsentation in eine dritte Repräsentation im Ortsraum, |
| (180) | Ausgeben und/oder Speichern der dritten Repräsentation im Ortsraum und/oder Übermitteln der dritten Repräsentation im Ortsraum an ein separates Computersystem. |

Fig. 2 zeigt beispielhaft und schematisch ein Verfahren zum Trainieren des Modells des maschinellen Lernens der vorliegenden Offenbarung.

Das Modell (MLM) des maschinellen Lernens wird auf Basis von Trainingsdaten trainiert. Die Trainingsdaten umfassen, für jede Referenzperson einer Vielzahl an Referenzpersonen, (i) eine erste Referenz-Differenzrepräsentation (RR_{D}¹) als Eingabedaten und (ii) eine zweite Referenz-Differenzrepräsentation (RR_{D}²) als Zieldaten.

In Fig. 2 ist nur ein Datensatz einer Referenzperson umfassend eine erste Referenz-Differenzrepräsentation (RR_{D}¹) und eine zweite Referenz-Differenzrepräsentation (RR_{D}²) dargestellt. Üblicherweise umfassen die Trainingsdaten eine Vielzahl solcher Datensätze.

Während des Trainings wird die erste Referenz-Differenzrepräsentation (RR_{D}¹) dem Modell (MLM) des maschinellen Lernens zugeführt. Das Modell ist konfiguriert und wird trainiert, auf Basis der ersten Referenz-Differenzrepräsentation (RR_{D}¹) und auf Basis von Modellparametern (und optional auf Basis weiterer Eingabedaten) eine vorhergesagte zweite Referenz-Differenzrepräsentation (RR_{D}²*) zu erzeugen. Die vorhergesagte zweite Referenz-Differenzrepräsentation (RR_{D}²*) wird mit der zweiten Referenz-Differenzrepräsentation (RR_{D}²) verglichen. Eine Fehlerfunktion (LF) wird verwendet, um Abweichungen zwischen der vorhergesagten zweiten Referenz-Differenzrepräsentation (RR_{D}²*) und der zweiten Referenz-Differenzrepräsentation (RR_{D}²) zu quantifizieren. In einem Optimierungsverfahren (z.B. einem Gradientenabstiegsverfahren) werden die Abweichungen durch Modifizieren der Modellparameter (MP) reduziert.

Die in Fig. 2 gezeigten Schritte werden für eine Vielzahl an Datensätzen durchgeführt. Das Training des Modells (MLM) des maschinellen Lernens kann beendet werden, wenn ein Stoppkriterium erfüllt ist. Ein solches Stoppkriterium kann beispielsweise sein: eine vorab festgelegte maximale Anzahl von Trainingsschritten/-zyklen/-epochen wurde durchgeführt, Abweichungen zwischen der vorhergesagten zweiten Referenz-Differenzrepräsentation und der zweiten Referenz-Differenzrepräsentation können nicht mehr durch Modifizieren der Modellparameter reduziert werden, ein vorab festgelegtes Minimum der Fehlerfunktion wird erreicht und/oder ein Extremwert (z. B. Maximum oder Minimum) eines anderen Leistungswerts wird erreicht.

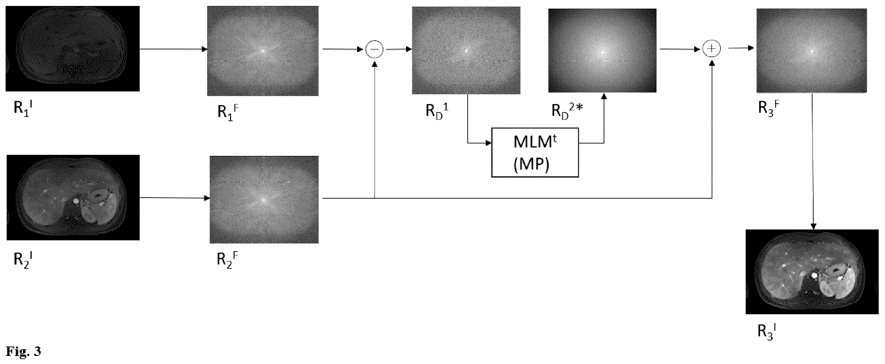

Ist das Training beendet, kann das trainierte Modell des maschinellen Lernens zur Vorhersage verwendet werden. Dies ist schematisch und beispielhaft in Fig. 3 dargestellt.

Fig. 2 zeigt neben dem Training des Modells (MLM) des maschinellen Lernens, wie die Trainingsdaten gewonnen werden können. Dies ist, wie das Training, anhand einer Referenzperson gezeigt.

Der in Fig. 2 gezeigte Ausgangspunkt sind drei Referenz-Repräsentationen, eine erste Referenz-Repräsentation (RR₁^{I}), eine zweite Referenz-Repräsentation (RR₂^{I}) und eine dritte Referenz-Repräsentation (RR₃^{I}).

Die erste Referenz-Repräsentation (RR₁^{I}) repräsentiert eine Leber oder einen Teil einer Leber der Referenzperson in einer portalvenösen Phase nach Applikation des hepatobiliären Kontrastmittels im Ortsraum. Die zweite Referenz-Repräsentation (RR₂^{I}) repräsentiert die Leber oder den Teil der Leber der Referenzperson in einer Übergangsphase nach Applikation des hepatobiliären Kontrastmittels im Ortsraum. Die dritte Referenz-Repräsentation (RR₃^{I}) repräsentiert die Leber oder den Teil der Leber der Referenzperson in einer hepatobiliären Phase nach Applikation des hepatobiliären Kontrastmittels im Ortsraum.

Aus der ersten Referenz-Repräsentation (RR₁^{I}) wird durch eine Transformation, z.B. eine Fourier-Transformation, eine erste Referenz-Repräsentation (RR₁^{F}) im Frequenzraum erzeugt. Die erste Referenz-Repräsentation (RR₁^{F}) repräsentiert die Leber oder den Teil der Leber der Referenzperson in der portalvenösen Phase nach Applikation des hepatobiliären Kontrastmittels im Frequenzraum.

Aus der zweiten Referenz-Repräsentation (RR₂^{I}) wird durch eine Transformation, z.B. eine Fourier-Transformation, eine zweite Referenz-Repräsentation (RR₂^{F}) im Frequenzraum erzeugt. Die zweite Referenz-Repräsentation (RR₂^{F}) repräsentiert die Leber oder den Teil der Leber der Referenzperson in der Übergangsphase nach Applikation des hepatobiliären Kontrastmittels im Frequenzraum.

Aus der dritten Referenz-Repräsentation (RR₃^{I}) wird durch eine Transformation, z.B. eine Fourier-Transformation, eine dritte Referenz-Repräsentation (RR₃^{F}) im Frequenzraum erzeugt. Die dritte Referenz-Repräsentation (RR₃^{F}) repräsentiert die Leber oder den Teil der Leber der Referenzperson in der hepatobiliären Phase nach Applikation des hepatobiliären Kontrastmittels im Frequenzraum.

Auf Basis der ersten Referenz-Repräsentation (RR₁^{F}) und der zweiten Referenz-Repräsentation (RR₂^{F}) wird die erste Referenz-Differenzrepräsentation (RR_{D}¹) erzeugt. Das Erzeugen der ersten Referenz-Differenzrepräsentation (RR_{D}¹) kann ein Subtrahieren der ersten Referenz-Repräsentation (RR₁^{F}) von der zweiten Referenz-Repräsentation (RR₂^{F}) umfassen. Das Erzeugen der ersten Referenz-Differenzrepräsentation (RR_{D}¹) kann ein Subtrahieren der zweiten Referenz-Repräsentation (RR₁^{F}) von der ersten Referenz-Repräsentation (RR₂^{F}) umfassen.

Auf Basis der zweiten Referenz-Repräsentation (RR₂^{F}) und der dritten Referenz-Repräsentation (RR₃^{F}) wird die zweite Referenz-Differenzrepräsentation (RR_{D}²) erzeugt. Das Erzeugen der zweiten Referenz-Differenzrepräsentation (RR_{D}²) kann ein Subtrahieren der zweiten Referenz-Repräsentation (RR₂^{F}) von der dritten Referenz-Repräsentation (RR₃^{F}) umfassen.

Ebenso ist es möglich, die zweite Referenz-Differenzrepräsentation (RR_{D}²) auf Basis der ersten Referenz-Repräsentation (RR₁^{F}) und der dritten Referenz-Repräsentation (RR₃^{F}) zu erzeugen. Das Erzeugen der zweiten Referenz-Differenzrepräsentation (RR_{D}²) kann ein Subtrahieren der ersten Referenz-Repräsentation (RR₂^{F}) von der dritten Referenz-Repräsentation (RR₃^{F}) umfassen.

In Fig. 2 ist dargestellt, wie die Ortsraum-Repräsentationen mittels Transformationen in die Frequenzraum-Repräsentationen überführt werden. Bei den Ortsraum-Repräsentationen handelt es sich üblicherweise um das Ergebnis einer radiologischen Untersuchung an der Referenzperson. Bei den Ortsraum-Repräsentationen handelt es sich also üblicherweise um messtechnisch erzeugte Repräsentationen. Es sei angemerkt, dass die Ortsraum-Repräsentationen nicht zwingend notwendig sind. Es ist ebenso möglich, dass die Frequenzraum-Repräsentationen als Ergebnis der radiologischen Untersuchung an der Referenzperson anfallen. Zum Beispiel werden im Rahmen einer MRT-Untersuchung üblicherweise so genannte k-Raum-Daten erzeugt, die eine Repräsentation eines Untersuchungsbereichs eines Untersuchungsobjekts im Frequenzraum sind. Es ist also beispielsweise möglich, dass die Frequenzraum-Repräsentationen direkt bei der radiologischen Untersuchung an der Referenzperson anfallen und zum Erzeugen der Trainingsdaten (erste Referenz- Differenzrepräsentation, zweite Referenz-Differenzrepräsentation) verwendet werden.

Fig. 3 zeigt beispielhaft und schematisch die Verwendung des trainierten Modells (MLM^{t}) des maschinellen Lernens zur Vorhersage.

In einem ersten Schritt werden eine erste Repräsentation (R₁^{F}) und eine zweite Repräsentation (R₁^{F}) bereitgestellt.

Die erste Repräsentation (R₁^{F}) repräsentiert eine Leber oder einen Teil einer Leber eines Menschen in einer portalvenösen Phase nach Applikation eines hepatobiliären Kontrastmittels im Frequenzraum.

Die zweite Repräsentation (R₂^{F}) repräsentiert die Leber oder den Teil der Leber des Menschen in einer Übergangsphase nach Applikation des hepatobiliären Kontrastmittels im Frequenzraum.

Die erste Repräsentation (R₁^{F}) im Frequenzraum wird auf Basis einer ersten Repräsentation (R₁^{I}) im Ortsraum erzeugt. Die erste Ortsraum-Repräsentation (R₁^{I}) repräsentiert die Leber oder den Teil der Leber des Menschen in der portalvenösen Phase nach Applikation des hepatobiliären Kontrastmittels im Ortsraum. Die erste Repräsentation (R₁^{F}) im Frequenzraum kann beispielsweise durch Fourier-Transformation aus der ersten Repräsentation (R₁^{I}) im Ortsraum erzeugt werden.

Die zweite Repräsentation (R₂^{F}) im Frequenzraum wird auf Basis einer zweiten Repräsentation (R₂^{I}) im Ortsraum erzeugt. Die zweite Ortsraum-Repräsentation (R₂^{I}) repräsentiert die Leber oder den Teil der Leber des Menschen in der Übergangsphase nach Applikation des hepatobiliären Kontrastmittels im Ortsraum. Die zweite Repräsentation (R₂^{F}) im Frequenzraum kann beispielsweise durch Fourier-Transformation aus der zweiten Repräsentation (R₂^{I}) im Ortsraum erzeugt werden.

Auf Basis der ersten Repräsentation (R₁^{F}) im Frequenzraum und der zweiten Repräsentation (R₂^{F}) im Frequenzraum wird eine erste Differenzrepräsentation (R_{D}¹) erzeugt. Die erste Differenzrepräsentation (R_{D}¹) repräsentiert eine Differenz zwischen der ersten Repräsentation (R₁^{F}) und der zweiten Repräsentation (R₂^{F}). Das Erzeugen der ersten Differenzrepräsentation (R_{D}¹) kann beispielsweise das Subtrahieren der ersten Repräsentation (R₁^{F}) von der zweiten Repräsentation (R₂^{F}) umfassen. Das Erzeugen der ersten Differenzrepräsentation (R_{D}¹) kann beispielsweise das Subtrahieren der zweiten Repräsentation (R₂^{F}) von der ersten Repräsentation (R₁^{F}) umfassen. Hier wird diejenige Subtraktion gewählt, die auch beim Trainieren des Modells des maschinellen Lernens gewählt wurde.

Die erste Differenzrepräsentation (R_{D}¹) wird dem trainierten Modell (MLM^{t}) des maschinellen Lernens zugeführt. Das Modell (MLM^{t}) des maschinellen Lernens kann beispielsweise wie in Bezug zu Fig. 2 beschrieben trainiert worden sein.

Das trainierte Modell (MLM^{t}) des maschinellen Lernens erzeugt auf Basis der ersten Differenzrepräsentation (R_{D}¹) (und optional auf Basis weiterer Eingabedaten) eine vorhergesagte zweite Differenzrepräsentation (R_{D}²*).

In einem weiteren Schritt wird eine dritte Repräsentation (R₃^{F}) erzeugt. Das Erzeugen der dritten Repräsentation (R₃^{F}) kann ein Addieren der zweiten Repräsentation (R₂^{I}) zu der vorhergesagten zweiten Differenzrepräsentation (R_{D}²*) umfassen. Das Erzeugen der dritten Repräsentation (R₃^{F}) kann ein Addieren der ersten Repräsentation (R₁^{F}) zu der vorhergesagten zweiten Differenzrepräsentation (R_{D}²*) umfassen. Hier wird diejenige Addition gewählt, die auch beim Trainieren des Modells des maschinellen Lernens gewählt wurde.

Die dritte Repräsentation (R₃^{F}) repräsentiert die Leber oder den Teil der Leber des Menschen in einer hepatobiliären Phase nach Applikation des hepatobiliären Kontrastmittels im Frequenzraum. Auf Basis der dritten Repräsentation (R₃^{F}) im Frequenzraum kann durch eine Transformation, z.B. eine inverse Fourier-Transformation eine dritte Repräsentation (R₃^{I}) im Ortsraum erzeugt werden. Die dritte Ortsraum-Repräsentation (R₃^{I}) repräsentiert die Leber oder den Teil der Leber des Menschen in der hepatobiliären Phase nach Applikation des hepatobiliären Kontrastmittels im Ortsraum.

Die dritte Ortsraum-Repräsentation (R₃^{I}) kann ausgegeben, d.h. z.B. auf einem Monitor angezeigt und/oder auf einem Drucker ausgedruckt und/oder in einem Datenspeicher gespeichert und/oder an ein separates Computersystem übermittelt werden.

In Fig. 3 ist dargestellt, wie die Ortsraum-Repräsentationen mittels Transformationen in die Frequenzraum-Repräsentationen überführt werden. Bei den Ortsraum-Repräsentationen handelt es sich üblicherweise um das Ergebnis einer radiologischen Untersuchung an dem Menschen. Bei den Ortsraum-Repräsentationen handelt es sich also üblicherweise um messtechnisch erzeugte Repräsentationen. Es sei angemerkt, dass die Ortsraum-Repräsentationen nicht zwingend notwendig sind. Es ist ebenso möglich, dass die Frequenzraum-Repräsentationen als Ergebnis der radiologischen Untersuchung an dem Menschen anfallen. Zum Beispiel werden im Rahmen einer MRT-Untersuchung üblicherweise so genannte k-Raum-Daten erzeugt, die eine Repräsentation eines Untersuchungsbereichs eines Untersuchungsobjekts im Frequenzraum sind. Es ist also beispielsweise möglich, dass die Frequenzraum-Repräsentationen direkt bei der radiologischen Untersuchung an dem Menschen anfallen und zum Erzeugen der Eingabe (erste Differenzrepräsentation) für das trainierte Modell des maschinellen Lernens verwendet werden.

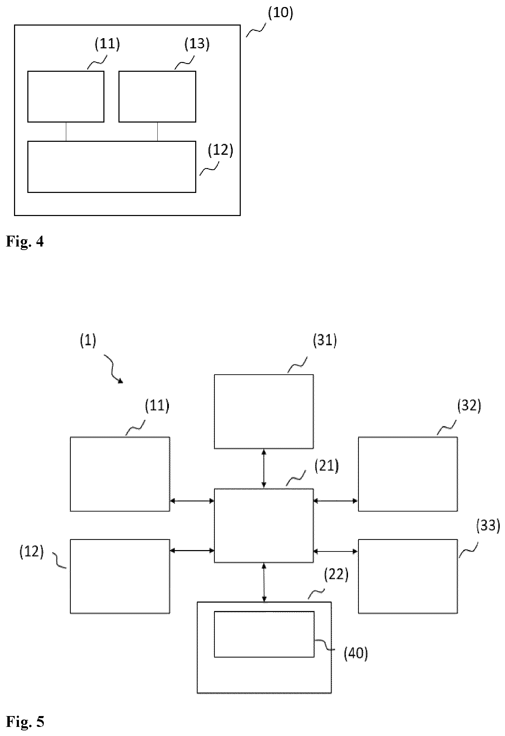

Fig. 4 zeigt beispielhaft und schematisch ein Computersystem gemäß der vorliegenden Offenbarung, das zum Trainieren des Modells des maschinellen Lernens und/oder zur Verwendung des trainierten Modells des maschinellen Lernens zur Vorhersage verwendet werden kann.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 4 gezeigte Computersystem (10) umfasst eine Empfangseinheit (11), eine Steuer- und Recheneinheit (12) und eine Ausgabeeinheit (13).

Die Steuer- und Recheneinheit (12) dient der Steuerung des Computersystems (10), der Koordinierung der Datenflüsse zwischen den Einheiten des Computersystems (10) und der Durchführung von Berechnungen.

Die Steuer- und Recheneinheit (12) ist konfiguriert:
- eine erste Repräsentation bereitzustellen oder die Empfangseinheit zu veranlassen, die erste Repräsentation zu empfangen, wobei die erste Repräsentation eine Leber oder einen Teil einer Leber eines Menschen in einer portalvenösen Phase nach Applikation eines hepatobiliären Kontrastmittels im Frequenzraum repräsentiert,
- eine zweite Repräsentation bereitzustellen oder die Empfangseinheit zu veranlassen, die zweite Repräsentation zu empfangen, wobei die zweite Repräsentation die Leber oder den Teil der Leber des Menschen in einer Übergangsphase nach Applikation des hepatobiliären Kontrastmittels im Frequenzraum repräsentiert,
- eine erste Differenzrepräsentation auf Basis der ersten Repräsentation und der zweiten Repräsentation zu erzeugen, wobei die erste Differenzrepräsentation eine Differenz zwischen der ersten Repräsentation und der zweiten Repräsentation repräsentiert,
- die erste Differenzrepräsentation in ein trainiertes Modell des maschinellen Lernens einzugeben,
- eine zweite Differenzrepräsentation als Ausgabe von dem Modell des maschinellen Lernens zu empfangen,
- eine dritte Repräsentation zu erzeugen, wobei das Erzeugen der dritten Repräsentation ein Addieren der zweiten Differenzrepräsentation zu der zweiten Repräsentation oder zu der ersten Repräsentation umfasst,
- die dritte Repräsentation in eine dritte Repräsentation im Ortsraum zu transformieren,
- die Ausgabeeinheit zu veranlassen, die dritte Repräsentation im Ortsraum auszugeben, in einem Datenspeicher zu speichern und/oder an ein separates Computersystem zu übermitteln.

Fig. 5 zeigt beispielhaft und schematisch eine weitere Ausführungsform des erfindungsgemäßen Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 4 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das erfindungsgemäße Computersystem kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Gegenstand der vorliegenden Erfindung ist auch ein Computerprogrammprodukt. Ein solches Computerprogrammprodukt umfasst einen nichtflüchtigen Datenträger wie beispielsweise eine CD, eine DVD, ein USB-Stick oder ein anderes Medium zum Speichern von Daten. Auf dem Datenträger ist ein Computerprogramm gespeichert. Das Computerprogramm kann in einen Arbeitsspeicher eines Computersystems (insbesondere in einen Arbeitsspeicher eines Computersystems der vorliegenden Offenbarung) geladen werden und dort das Computersystem dazu veranlassen, folgende Schritte ausführen:
- Bereitstellen einer ersten Repräsentation, wobei die erste Repräsentation eine Leber oder einen Teil einer Leber eines Menschen in einer portalvenösen Phase nach Applikation eines hepatobiliären Kontrastmittels im Frequenzraum repräsentiert,
- Bereitstellen einer zweiten Repräsentation, wobei die zweite Repräsentation die Leber oder den Teil der Leber des Menschen in einer Übergangsphase nach Applikation des hepatobiliären Kontrastmittels im Frequenzraum repräsentiert,
- Erzeugen einer ersten Differenzrepräsentation auf Basis der ersten Repräsentation und der zweiten Repräsentation, wobei die erste Differenzrepräsentation eine Differenz zwischen der ersten Repräsentation und der zweiten Repräsentation repräsentiert,
- Eingeben der ersten Differenzrepräsentation in ein trainiertes Modell des maschinellen Lernens,
- Empfangen einer zweiten Differenzrepräsentation als Ausgabe von dem Modell des maschinellen Lernens,
- Erzeugen einer dritten Repräsentation, wobei das Erzeugen der dritten Repräsentation ein Addieren der zweiten Differenzrepräsentation zu der zweiten Repräsentation oder zu der ersten Repräsentation umfasst,
- Transformieren der dritten Repräsentation in eine dritte Repräsentation im Ortsraum,
- Ausgeben und/oder Speichern der dritten Repräsentation im Ortsraum und/oder Übermitteln der dritten Repräsentation im Ortsraum an ein separates Computersystem,
wobei das trainierte Modell des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation als Eingabedaten und (ii) eine zweite Referenz-Differenzrepräsentation als Zieldaten umfassen, wobei die erste Referenz-Differenzrepräsentation eine Differenz einer ersten Referenz-Repräsentation und einer zweiten Referenz-Repräsentation repräsentiert und die zweite Referenz-Differenzrepräsentation eine Differenz der zweiten Referenz-Repräsentation und einer dritten Referenz-Repräsentation oder der ersten Referenz-Repräsentation und der dritten Referenz-Repräsentation repräsentiert, wobei die erste Referenz-Repräsentation eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation die Leber oder den Teil der Leber der Referenzperson in einer Übergangsphase nach Applikation des hepatobiliären Kontrastmittels im Frequenzraum repräsentiert und die dritte Referenz-Repräsentation die Leber oder den Teil der Leber der Referenzperson in einer hepatobiliären Phase nach Applikation des hepatobiliären Kontrastmittels im Frequenzraum repräsentiert.

## Patentansprüche

1. Computer-implementiertes Verfahren umfassend die Schritte:
- Bereitstellen einer ersten Repräsentation (R₁^{F}), wobei die erste Repräsentation (R₁^{F}) eine Leber oder einen Teil einer Leber eines Menschen in einer portalvenösen Phase nach Applikation eines hepatobiliären Kontrastmittels im Frequenzraum repräsentiert,
- Bereitstellen einer zweiten Repräsentation (R₂^{F}), wobei die zweite Repräsentation (R₂^{F}) die Leber oder den Teil der Leber des Menschen in einer Übergangsphase nach Applikation des hepatobiliären Kontrastmittels im Frequenzraum repräsentiert,
- Erzeugen einer ersten Differenzrepräsentation (R_{D}¹) auf Basis der ersten Repräsentation (R₁^{F}) und der zweiten Repräsentation (R₂^{F}), wobei die erste Differenzrepräsentation (R_{D}¹) eine Differenz zwischen der ersten Repräsentation (R₁^{F}) und der zweiten Repräsentation (R₂^{F}) repräsentiert,
- Eingeben der ersten Differenzrepräsentation (R_{D}¹) in ein trainiertes Modell (MLM^{t}) des maschinellen Lernens,
- Empfangen einer zweiten Differenzrepräsentation (R_{D}²*) als Ausgabe von dem trainierten Modell (MLM^{t}) des maschinellen Lernens,
- Erzeugen einer dritten Repräsentation (R₃^{F}), wobei das Erzeugen der dritten Repräsentation (R₃^{F}) ein Addieren der zweiten Differenzrepräsentation (R_{D}²*) zu der zweiten Repräsentation (R₂^{F}) oder zu der ersten Repräsentation (R₁^{F}) umfasst,
- Transformieren der dritten Repräsentation (R₃^{F}) in eine dritte Repräsentation (R₃^{I}) im Ortsraum,
- Ausgeben und/oder Speichern der dritten Repräsentation (R₃^{I}) im Ortsraum und/oder Übermitteln der dritten Repräsentation (R₃^{I}) im Ortsraum an ein separates Computersystem,
wobei das trainierte Modell (MLM^{t}) des maschinellen Lernens in einem überwachten Lernverfahren auf Basis von Trainingsdaten trainiert wurde, wobei die Trainingsdaten für jede Referenzperson einer Vielzahl an Referenzpersonen (i) eine erste Referenz-Differenzrepräsentation (RR_{D}¹) als Eingabedaten und (ii) eine zweite Referenz-Differenzrepräsentation (RR_{D}²) als Zieldaten umfassen, wobei die erste Referenz-Differenzrepräsentation (RR_{D}¹) eine Differenz einer ersten Referenz-Repräsentation (RR₁^{F}) und einer zweiten Referenz-Repräsentation (RR₂^{F}) repräsentiert und die zweite Referenz-Differenzrepräsentation (RR_{D}²) eine Differenz der zweiten Referenz-Repräsentation (RR₂^{F}) und einer dritten Referenz-Repräsentation (RR₃^{F}) oder der ersten Referenz-Repräsentation (RR₁^{F}) und der dritten Referenz-Repräsentation (RR₃^{F}) repräsentiert, wobei die erste Referenz-Repräsentation (RR₁^{F}) eine Leber oder einen Teil einer Leber der Referenzperson in einer portalvenösen Phase nach Applikation des hepatobiliären Kontrastmittels im Frequenzraum repräsentiert, wobei die zweite Referenz-Repräsentation (RR₂^{F}) die Leber oder den Teil der Leber der Referenzperson in einer Übergangsphase nach Applikation des hepatobiliären Kontrastmittels im Frequenzraum repräsentiert und die dritte Referenz-Repräsentation (RR₃^{F}) die Leber oder den Teil der Leber der Referenzperson in einer hepatobiliären Phase nach Applikation des hepatobiliären Kontrastmittels im Frequenzraum repräsentiert.

2. Verfahren gemäß Anspruch 1, wobei das Erzeugen der ersten Differenzrepräsentation (R_{D}¹) ein Subtrahieren der ersten Repräsentation (R₁^{F}) von der zweiten Repräsentation (R₂^{F}) umfasst.

3. Verfahren gemäß einem der Ansprüche 1 oder 2, wobei das Erzeugen der dritten Repräsentation (R₃^{F}) ein Addieren der zweiten Differenzrepräsentation (R_{D}²*) zu der zweiten Repräsentation (R₂^{F}) umfasst, und wobei die zweite Referenz-Differenzrepräsentation (RR_{D}²) auf Basis der zweiten Referenz-Repräsentation (RR₂^{F}) und der dritten Referenz-Repräsentation (RR₃^{F}) erzeugt wurde, wobei das Erzeugen der zweiten Referenz-Differenzrepräsentation (RR_{D}²) ein Subtrahieren der zweiten Referenz-Repräsentation (RR₂^{F}) von der dritten Referenz-Repräsentation (RR₃^{F}) umfasste.

4. Verfahren gemäß einem der Ansprüche 1 bis 3, wobei die erste Repräsentation (R₁^{F}) und die zweite Repräsentation (R₂^{I}) das Ergebnis einer radiologischen Untersuchung an dem Menschen ist.

5. Verfahren gemäß einem der Ansprüche 1 bis 3, ferner umfassend:
- Erzeugen der ersten Repräsentation (R₁^{F}) aus einer ersten Ortsraum-Repräsentation (R₁^{I}), wobei die erste Ortsraum-Repräsentation (R₁^{I}) die Leber oder den Teil der Leber des Menschen in der portalvenösen Phase nach Applikation des hepatobiliären Kontrastmittels im Ortsraum repräsentiert,
- Erzeugen der zweiten Repräsentation (R₂^{I}) aus einer zweiten Ortsraum-Repräsentation (R₂^{I}), wobei die zweite Ortsraum-Repräsentation (R₂^{I}) die Leber oder den Teil der Leber des Menschen in der Übergangsphase nach Applikation des hepatobiliären Kontrastmittels im Ortsraum repräsentiert,
wobei die erste Ortsraum-Repräsentation (R₁^{I}) und die zweite Ortsraum-Repräsentation (R₂^{I}) das Ergebnis einer radiologischen Untersuchung an dem Menschen ist.

6. Verfahren gemäß einem der Ansprüche 1 bis 4, wobei die erste Referenz-Repräsentation (RR₁^{F} und die zweite Referenz-Repräsentation (RR₂^{F}) einer jeden Referenzperson das Ergebnis einer radiologischen Untersuchung an der Referenzperson ist.

7. Verfahren gemäß Anspruch 5, wobei die erste Referenz-Repräsentation (RR₁^{F} im Frequenzraum auf Basis einer ersten Referenz-Repräsentation (RR₁^{I}) im Ortsraum erzeugt wurde, wobei die erste Referenz-Repräsentation (RR₁^{I}) im Ortsraum die Leber oder den Teil der Leber der Referenzperson in der portalvenösen Phase nach Applikation des hepatobiliären Kontrastmittels im Ortsraum repräsentiert, wobei die zweite Referenz-Repräsentation (RR₂^{F}) im Frequenzraum auf Basis einer zweiten Referenz-Repräsentation (RR₂^{I}) im Ortsraum erzeugt wurde, wobei die zweite Referenz-Repräsentation (RR₂^{I}) im Ortsraum die Leber oder den Teil der Leber der Referenzperson in der Übergangsphase nach Applikation des hepatobiliären Kontrastmittels im Ortsraum repräsentiert,
wobei die dritte Referenz-Repräsentation (RR₃^{F}) im Frequenzraum auf Basis einer dritten Referenz-Repräsentation (RR₃^{I}) im Ortsraum erzeugt wurde, wobei die dritte Referenz-Repräsentation (RR₃^{I}) im Ortsraum die Leber oder den Teil der Leber der Referenzperson in der hepatobiliären Phase nach Applikation des hepatobiliären Kontrastmittels im Ortsraum repräsentiert,
wobei die erste Referenz-Repräsentation (R₁^{I}) im Ortsraum und die zweite Referenz-Repräsentation (RR₂^{I}) im Ortsraum und die dritte Referenz-Repräsentation (RR₃^{I}) im Ortsraum einer jeden Referenzperson das Ergebnis einer radiologischen Untersuchung an der Referenzperson ist.

8. Verfahren gemäß einem der Ansprüche 4 bis 7, wobei die radiologische Untersuchung eine MRT-Untersuchung ist oder umfasst.

9. Verfahren gemäß einem der Ansprüche 4 bis 8, wobei die radiologische Untersuchung eine CT-Untersuchung ist oder umfasst.

10. Verfahren gemäß einem der Ansprüche 1 bis 9, wobei das hepatobiliäre Kontrastmittel ein MRT-Kontrastmittel ist oder umfasst.

11. Verfahren gemäß einem der Ansprüche 1 bis 10, wobei das hepatobiliäre Kontrastmittel einen oder mehrere der folgenden Substanzen umfasst:
- Gadoxetat-Dinatrium,
- Gd³⁺-Komplex einer Verbindung der Formel (I) 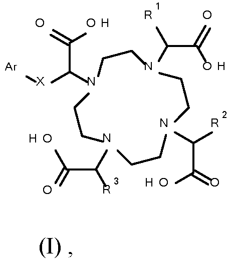 umfasst, wobei
Ar eine Gruppe ausgewählt aus 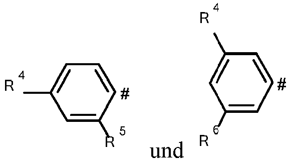 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-# ausgewählt wird,
wobei * die Anknüpfung zu Ar darstellt und # die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon,
- Gd³⁺-Komplex einer Verbindung der Formel (II) 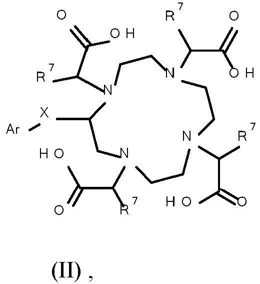 umfasst, wobei
Ar eine Gruppe ausgewählt aus 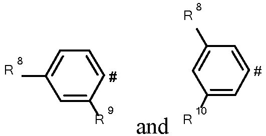 darstellt,
wobei # die Anknüpfung zu X darstellt,
X eine Gruppe darstellt, die aus CH₂, (CH₂)₂, (CH₂)₃, (CH₂)₄ and *-(CH₂)₂-O-CH₂-# ausgewählt wird, wobei * die Anknüpfung zu Ar darstellt und # die Anknüpfung zum Essigsäurerest darstellt,
R⁷ ein Wasserstoffatom oder eine Gruppe ausgewählt aus C₁-C₃-Alkyl, -CH₂OH,
-(CH₂)₂OH und -CH₂OCH₃ darstellt;
R⁸ eine Gruppe ausgewählt aus
C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon,
- Gadolinium 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat),
- Gadolinium 2,2',2"-{10-[(1S)-4-(4-butoxyphenyl)-1-carboxybutyl]-1,4,7,10-tetraazacyclododecan-1,4,7-triyl}triacetat.

12. Verfahren gemäß einem der Ansprüche 1 bis 11, wobei das trainierte Modell (MLM^{t}) des maschinellen Lernens ein künstliches neuronales Netzwerk ist oder umfasst.

13. Verfahren gemäß einem der Ansprüche 1 bis 12, wobei das Trainieren des Modells (MLM) des maschinellen Lernens umfasst:
für jede Referenzperson der Vielzahl an Referenzpersonen:
- Eingeben der ersten Referenz-Differenzrepräsentation (RR_{D}¹) in das Modell (MLM) des maschinellen Lernens,
- Empfangen einer vorhergesagten zweiten Referenz-Differenzrepräsentation (RR_{D}²*) als Ausgabe von dem Modell (MLM) des maschinellen Lernens,
- Quantifizieren einer Abweichung zwischen der zweiten Referenz-Differenzrepräsentation (RR_{D}²) und der vorhergesagten zweiten Referenz-Differenzrepräsentation (RR_{D}²*),
- Reduzieren der Abweichung durch Modifizieren von Modellparametern (MP) des Modells (MLM) des maschinellen Lernens.

14. Computersystem umfassend
• eine Empfangseinheit,
• eine Steuer- und Recheneinheit und
• eine Ausgabeeinheit,
wobei die Steuer- und Recheneinheit konfiguriert ist, das Verfahren gemäß einem der Ansprüche 1 bis 13 auszuführen.

15. Computerprogrammprodukt umfassend ein Computerprogramm, das in einen Arbeitsspeicher eines Computersystems geladen werden kann und dort das Computersystem dazu veranlasst, das Verfahren gemäß einem der Ansprüche 1 bis 13 auszuführen.

16. Kit umfassend ein hepatobiliäres Kontrastmittel und ein Computerprogrammprodukt gemäß Anspruch 15.
